# EUROPEAN PATENT SPECIFICATION

(11) **EP 1 343 472 B1**
(45) Date of publication and mention of the grant of the patent: **24.05.2006**
(21) Application number: 01985877.8
(22) Date of filing: 13.12.2001
(51) Int. Cl.: A61K 9/00, A61K 9/06

(54) **THIXOTROPIC NASAL SPRAY**
THIXOTROPES NASENSPRAY
AEROSOL NASAL THIXOTROPIQUE

(30) Priority: 23.12.2000 DE 10064950
(43) Date of publication of application: 17.09.2003
(73) Proprietor: analyze & realize ag, 13467 Berlin (DE)
(72) Inventor: JAENICKE, Christof, 13465 Berlin (DE)
(74) Representative: Patent- und Rechtsanwaltssozietät Maucher, Börjes & Kollegen
(86) International application number: PCT/EP2001/014655
(87) International publication number: WO 2002/051379

(56) References cited:
- EP-A- 0 733 357
- WO-A-00/47184
- WO-A-01/51014
- WO-A-91/06283

## Description

### Summary of the invention

The invention relates to the use of a thixotropic preparation free of active ingredients (active substances) for the manufacture of a product for the therapeutic treatment of nasal disorders, as described in claim 1 and under "Detailed description of the invention" below.

### Background to the invention

Numerous organic compounds, in particular with larger molar mass, and many inorganic compounds, in suitable dispersion media and, if required, with the use of specific methods, form colloidal solutions, known as sols. The stability of organic sols in particular, but also of a series of inorganic sols, depends primarily on the solvate sheath, which is bonded firmly to the surface of the colloid particle and holds it in solution. These sols are designated as hydrophilic or lyophilic. Under certain conditions their viscosity may become so great that a gelatinous mass is formed (coagulation), although the volume of the dispersion agent is predominant by far in comparison with the dispersion phase. These gelatinous masses, deriving from sols, are referred to as gels. It is possible, for example, by warming up, to reverse the formation of the gel and revert to the sol state (peptisation).

One particular type of conversion of sols into gels and vice-versa is thixotropy. Certain sols become gelatinous rapidly, if they are allowed to stand quietly. Under the effect of mechanical forces (shear forces), for example due to shaking, pressing through a nozzle, or stirring, the viscosity of these gels will be reduced to such an extent, however, that they must again be regarded as sols. If the system subjected to shear forces is again allowed to stand in peace, the high initial viscosity will within a short time be restored, and the specimen will again become a gel. The sol-gel conversion and vice-versa can be repeated as often as may be wished. This can be demonstrated very illustratively with a sol of magnesium trisilicate. Thixotropic processing is based on the fact that the dispersal phase, when standing, acquires a certain scaffold structure due to the forces of attraction between the particles, which is, however, so labile that it will at least in part be destroyed again by mechanical vibrations. In the resting phase, the scaffold structures are restored, and the viscosity rises again.

Thixotropic gels have already been applied in a series of formulations, in which situation, however, only their use in the cosmetics sector or, in the pharmaceutical sector, their function as a depot for pharmaceutical active substances are described. Examples of nasal formulations can be found in the International Application WO 98/00178, where the thixotropic gels are described as suspending media for solid particles of certain specific medicamentously active substances, due to their depot effect for these substances on the nasal mucous membrane, and in the International Application WO 94/05330, where likewise thixotropic basic formulations are used for the formulation of certain specific pharmaceutical active substances, with the aim of reducing their flowback and outflow from the nasal area, and so provide for a longer dwell time of the active substances in the nose. In both Applications, the gels contain constitutively specific medications (active substances). Other prior art also mentions compositions that may be or are thixotropic. WO 00/47184 relates to skin-sanitizing compositions which however comprise an alcohol antiseptic. WO 01/51014 mentions compositions for mucosa treatment, inter alia the nose, but only either as a carrier for or as a composition comprising a biologically active substance, e.g. a. cortioosteroid. WO 91/06283 mentions the moisturizing of skin or mucosa and does not mention an oily component. In EP 0 733 357, the semi-solid system always is the basis for an actual pharmaceutical with an active substance, such as ketoprofen.

The causes of allergic rhinitis, a disorder of the nasal mucous membrane, are in principle known. Mainly, an immune reaction takes place, which, due to specific mediators, incurs local and systemic effects in the patient. Primarily, tissue mast cells are attacked, which feature, *inter alia,* non-specific and specific receptors in the outer cell membrane (for immunoglobulin E, for example). The bonding of allergens to these receptors leads to the release of histamine. and other inflammatory mediators, such as leucotrienes and prostaglandins. As a consequence of the effect of these mediators, the musculature of the vessels becomes slack, the permeability of the vessels and the membrane increases, and constriction of the bronchial musculature occurs. The symptoms of allergic rhinitis follow from these reactions: Local rubor and swelling of the nasal mucosa and of the conjunctiva, as well as increased emergence of fluid into the tissue and resultant increased secretion in the nose and eyes. Systemic effects may also occur, such as hypotonic states in the cardiovascular system.

This cascade of effects is triggered primarily by exogenic agents, such as pollen, house dust, animal hair, foodstuff constituents, pharmaceutical preparations or mites, or by antibodies which were themselves formed in the organism as a reaction to pollen or other foreign substances. This is a disorder which is very frequently encountered: A survey conducted in 1992/1993 of 2,400 school students indicated that 13.5 % had suffered from hayfever in the previous year. Pollen allergies are the leaders in atopic disorders.

The therapy for allergic rhinitides, in particular for allergic rhinoconjunctivitis (pollinosis, "hayfever"), with aqueous nasal secretion, blocked respiration due to obstruction, itching nose, sneezing attacks combined with inflammation and itching irritation of the eyes, as well as tear flow, in addition, frequently, a general feeling of malaise, weakness, fatigue, exhaustion, in times of high allergy burden up to the level of complaints in other organ systems, bronchial hyperreactivity with asthma symptoms, exacerbation (worsening) of atopical dermatitis, or also gastrointestinal disorders) is based on three main principles: Allergen restriction, medicamentous therapy (symptomatic only), and specific immunotherapy (hyposensitisation).

Allergen restriction, which takes causal effect, is intended to avoid or reduce the exposure of the patient to allergens, in order to avoid contact with mast cells. This includes measures in the everyday conduct of life, such as keeping windows and doors closed, fitting pollen filters in air-conditioning systems, no sports practised in the open air, holidays in other climatic zones, etc. This however requires a high level of compliance on the part of the patient and his surroundings. The method is effective prophylactically , especially. In medication therapy, modern oral and topical pharmaceutical products are used, such as disodium chromoglycate (DNCG) and Nedocromil for topical administration, systemic or topical antihistamines, systemic or topical glucocorticoids, and α-sympathicomimetics and anticholinergics. DNCG, for example, has a stabilizing effect on the mast cells and prevents degranulation and mediator secretion. Its use must therefore be prophylactic, and it must be administered four times daily. The therapeutic effect is not immediate but takes place only with some hours of delay. Even in the event of slight symptoms, combinations of different medications are, as a rule, used nowadays. Concludingly, hyposensitisation is effected by regular injection of preparations which contain allergen extracts, and is based on the education of the immune system to a moderate reaction.

Medicamentous therapy is considerably elaborate and expensive, and as a general rule the attempt must also be made to reduce allergen exposure. Oversensitivity reactions are possible. The causally effective hyposensitisation requires in particular a high level of diagnostic effort, is elaborate, and (because of the regular injections over an extended period) is a burden on the patient and does not demonstrate any sustained or reliably reproducible effect.

German patent application DE 42 27 887 relates to the use at room temperature of gel-type mixtures of predominantly saturated hydrocarbons, such as vaseline, in the form of a nasal ointment, by application on parts of the nasal mucous membrane, in particular on the inner wall of the upper part of the nasal vestibule, as prophylaxis for inhalation-allergic reactions such as atopic rhinitis. Disadvantages are, for example, the somewhat uncomfortable administration (application using a small cotton rod), and the fact that only the front part of the nasal mucous membrane can be protected. This means that only incomplete coverage of the nasal mucous membrane is provided, and therefore no complete protection against contact of the allergens with the nasal mucous membrane and therefore with the mast cells is achieved. A corresponding vaseline product (Simarolin® ) is commercially available.

Against this background, the objective of the present invention is to provide new preparations which make additional forms of therapy possible, which show, as far as possible, low side-effects and which are effective in a simple manner (in particular prophylactically), and which make possible in particular a complete or extensive allergen restriction.

### General description of the invention

It has now been found, surprisingly, that the administration of thixotropic gels provides a positive effect against nasal disorders, and against allergic rhinitis in particular, without the addition of pharmaceutical active substances. The effect takes place locally and in the absence of any pharmaceutically active substances which could have local side-effects and, due to resorption, also systemic side-effects, which in this way are avoided. In the preferred embodiment, the high costs of the corresponding pharmaceutical active substances can be avoided. Without wishing to be bound solely to this explanation, it appears that the effect against the allergens is based on a "mechanical" blocking function (in particular due to the oily components contained and/or the viscosity of the gel), the allergens being no longer able to proliferate directly on the nasal mucous membrane due to the largely uniform distribution of the gel on the mucous membrane, and they therefore do not come in contact with the mast cells. As a result, the allergic reactions described hereinbefore are eliminated entirely or diminished. The blocking function takes place directly after administration.

A general advantage of the thixotropic gels is their easy use - for example, in contrast to thermosensitive gels no defined temperatures are required before administration. Simple shaking allows to bring them into the sol state appropriate for administration.

### Detailed description of the invention

The invention relates to the use of a thixotropic preparation, defined in greater detail hereinafter, which is free of active substances, for the manufacture of a pharmaceutical (or medical) product for the therapeutic treatment of nasal disorders in particular, and/or a method for the therapeutic treatment of nasal disorders, in particularthixotropic nose drops, free of active substances, especially a nasaly spray free of active substances, where the term "therapeutic treatment" also includes prophylaxis,
where the thixotropic preparation consists of
(A) an aqueous basis, whereby the aqueous phase can contain one or more additives in the form of one or more di- or polyols;
(B) an oily component; and
(C) one or more gel formers for thixotropic gels;
with or without the presence of further additives selected from the group consisting of binders, surfactants, preservation agents, colouring agents, flavouring substances, pH regulators, and regulators of the osmotic activity,
whereby, if salt-forming groups are present in the components, these may also be present in whole or in part as salts.

The terms generally used hereinbefore and hereinafter have for preference the meanings indicated below, unless indicated otherwise, whereby more specific meanings may be used independently of one another in preferred embodiments of the present inventions instead of the general definitions, these more specific significances describing especially preferred embodiments of the invention.

Where the term "at least one" or "one or more" occurs hereinbefore and hereinafter, this signifies in particular one to ten, for preference one to three, and in particular one or, further, two of the features enumerated, such as components. Where ranges are indicated, such as weight percentage ranges, these include the limit values indicated; thus, for example, "between X and Y" signifies "from and including X up to and including Y".

Fractions of components are indicated as "% by weight" (percentage by weight), related to the finished (complete) preparation.

"Free of active substances", "without the addition of pharmaceutical(ly) active substances" or the like signifies that the preferred preparation according to the invention is free of any added pharmaceutical active substances, such as, in particular, bronchodilators, antipyretics, analgetics, antiphlogistics, antiarrhythmics, blood-pressure reducing agents, vasodilators, anticholinergics, antiarteriosclerotics, enzymes, antibodies, secretolytics, ulcer preparations, antiproliferative agents, vasoconstrictors, expectorants, antitussiva, mucolytics, or secretomotorics; in particular, free of antiallergics (including those referred to hereinbefore), such as α-sympathicometics (in particular Phenylephrin, Ephedrin, Tetryzolin, Naphazolin, Oxymetozolin, Xylometazolin or Tramazolin), antihistamines, non-steroidal or steroidal anti-inflammatory active substances (in particular Triamcinolone acetonide, glucocorticoids, such as Prednisolone, Triamcinolonacetonide, Clomethasone, Dexamethasone, or Fluticasone), β₂ sympathomimetics; and mast cell stabilizers, or the like; pharmaceutically acceptable salts thereof, if salt-forming groups are present; or combinations of two or more of the aforementioned active substances or their pharmaceutically acceptable salts. The principle of the invention is exactly that the preparation according to the invention already achieves a therapeutic (especially prophylactic) effect even without such active substances.

Further examples for active substances to be excluded are those named in WO 98/00178 and WO 94/05330, and these two applications are therefore incorporated in particular by reference herein, in particular in this respect; for preference, these active substances in addition include etheric oils and plant extracts, such as menthol, eucalyptol, camphor, or extracts of rosemary, aniseed, coriander, lovage, peppermint, rosemary, thyme, or basil (these may however be used purely as flavouring substances, but are preferably absent); α-sympathomimetics, in particular Indanazolin, Etitefrin, or Fenoxazolin; antihistamines, in particular Azelastin, Levocabastin, Cetirizin, Loratidin, Terfenadin or Fexofenadin; leucotriene antagonists (especially preferred), such as Prankulast, Zafirlukast, or Montelukast; certain corticoids, in particular Budensonide, Flunisolide, Fluocortinbutyl, Fluticason-17-propionate, Mometasone, Mometasone Furoate, or allergen extracts; or antibiotics; or, provided that salt-forming groups are present, pharmaceutically acceptable salts of the aforesaid substances and compounds; or mixtures thereof.

Mast cell stabilizers are especially cromoglycinic acid or the salts thereof, in particular sodium cromoglycate, or Nedocromil.

The term "product" is to be understood to mean in particular a pharmaceutical product in the sense of a formulation, for preference a pharmaceutical product, whereby this term is not restricted to pharmaceutical products suitable for registration, or a medical product or fictitious pharmaceuticals ("Geftungsarzneimittel", that is, products which the German Drug Law subsumes under medical products, though they do not fall under the strict term "Arzneimittel" (drugs)).

The term "thixotropic preparation" is to be understood to mean such that feature, when subjected to shear forces (shaking, pressing through a nozzle, stirring, or the like), a low viscosity (sol state), suitable in particular for use as nose drops or in particular as a nasal spray, for preference in the range from 1 to 40, in particular from 5 to 20 Pa*s (= kg/(m x s)), while by contrast when in a state of rest feature a high viscosity, for preference in the range from 40 to 100 Pa*s. The measurement is effected by means of a rotation viscosimeter.

The thixotropic preparation comprises at least three components: An aqueous basis, at least one oily component (which is a very important component), and at least one gel-forming agent for thixotropic gels (components having the effect of creating suspensions, which may lend the preparation thixotropic properties). With particular preference, just these three components are present.

The components referred to hereinbefore and hereinafter are in particular selected from among those such as are listed in pharmacopoeia, e.g. in the US Pharmacopoeia National Formulary, the Pharmacopoea Europea, the Pharmacopoea Helvetica, the British Pharmacopoeia, or the German Pharmacopoeia, or supplements, such as by way of decrees.

As an aqueous basis, water is particularly suitable, to which (already in the aqueous basis as such, in mixture with other constituents of a preparation according to the invention, or as a constituent of the finished preparation) as other water-soluble components diols or polyols can be added, in particular ethylene glycol, 1,2 propylene glycol, a sugar alcohol such as sorbite, hexite, or mannite, or in particular glycerine, as additives (e.g. supporting and stabilizing the formation of the gel). Water is present for preference in a proportion of 10 to 80, for preference from 30 to 70, e.g. 40 to 60 % by weight, and stabilizing additives may be contained in this component in a quantity in the range from, for preference, 0.1 to 10 % by weight, related to the finished composition, respectively. The water proportion is suitable in particular for regulating the basic viscosity of the thixotropic gel.

A large number of products are suitable as oily components, in particular:
(i) Hydrocarbons, e.g. mineral oils, in particular paraffin, paraffin oil (in particular white paraffin oil, low-viscosity or in a broader embodiment high-viscosity paraffin oil), purcellin oil or perhydrosqualene, or further hard paraffin or vaseline, whereby low-visoosity paraffin oil is highly preferred; or
(ii) Vegetable oils, e.g. almond oil, groundnut oil, wheatgerm oil, rape oil, linseed oil, apricot oil, walnut oil, palm oil, pistachio oil, sesame oil, poppyseed oil, pine oil, castor oil, soya oil, avocado oil, cocoa oil, hazelnut oil, olive oil, grapeseed oil, rice oil, maize germ oil, peach-kernel oil, coffee oil, Jojoba oil, sunflower oil, thistle oil, cocoa butter or the like, or the hydrated, polyoxyethylated, polyoxy- or hydrated polyoxyderivatives or fractionated derivatives thereof, whereby castor oil is especially preferred;
or mixtures of two or more of these components.

Further examples of oily components with less preferred embodiments of the invention are animal oils, saturated or non-saturated esters, higher alcohols and/or silicone oils, or mixtures of two or more of these components.

To reinforce the retention on the mucous membranes, waxes, together with one or more oils such as those defined hereinbefore, may form part of an oily component of this type, such as camauba wax, Cera alba, Cera flava, Cera chinesis, Cera japonica, Candelilla wax, microcrystalline wax, wool wax or okozerite.

Particularly preferred are the oily components referred to under (i) and (ii), especially paraffin oil (in particular low-viscosity paraffin oil) or vegetable oils (in particular castor oil).

The oily component has for preference a proportion by weight in the finished preparation from 10 to 80, in particular from 40 to 70 % by weight.

As gel formers for thixotropic gels consideration may be given to, for example:
(a) Organic suspension media, such as
   (i) polysaccharides, in particular cellulose (in particular microcrystalline cellulose, such as Avicel® (FMC Corporation, Philadelphia, USA) or cellulose derivatives, such as carboxymethyl cellulose or the salts thereof (in particular alkaline metal salts, such as sodium salts), alone or in mixtures with microcrystalline cellulose, methyl cellulose, guar gum, traganth or dextrine esters, such as is disclosed in EP 0 736 545, which is hereby incorporated by reference in this respect:
   (ii) polymer compounds which do not belong to the polysaccharides, in particular polyvinyl alcohols, polyacrylates, such as polyacrylic acid or polymethacrylate (cross-bonded in particular), polyacrylate block copolymers with alternating hydrophilic and hydrophobic blocks, such as, in particular, Hypane hydrogels (Kingston Technology Inc., N.Y., USA), such as in EP 0 836 847, which is hereby incorporated in this respect; or
   (iii) special organic gel formers, such as oxyethylenated (= polyoxyethylenated) polyol fatty acid esters, in particular alkyl- (especially methyl-) monosaccharide-C₆-C₂₄-fatty acid esters, or mixtures thereof (alone or in mixtures with appropriate non-oxyethylenated polyol-fatty acid esters, in particular alkyl- (especially methyl-) monosaccharide-C₆-C₂₄-fatty acid esters, or mixtures thereof, preferably methylglucoside-sesquistearate (= mixture of the mono and distearate), oxyethylenated with ethylene oxide (in particular 2 to 50, and especially 20 mol), with 20 mol ethylene oxide added, available for example under the brandname "Glucamate SSE 20® " from Amerchol Corp. (Du Pont), Edison, NJ, USA; CAS No. 68389-70-8) or a mixture thereof with methylglucoside-sesquistearate (available for example under the brandname "Glucamate SSE 20® " (Amerchol); CAS No. 68389-70-8)), for preference in the proportion by weight of 10:1 to 1:10, in particular from 3:1 to 1:3, e.g. of 1:1; others are the mixtures described in WO 94/14822 of metal salts of phosphoric acid diesters and organopolysiloxanes, which are derivatised with a polyvalent metal salt of a phosphoric acid diester containing silicon, which is why this patent application is hereby incorporated in this respect; or
(b) Inorganic suspension media, such as kaolin clays forming colloids, in particular (especially hydrated) metal oxide silicates, such as bentonite, or (for preference highly dispersed colloidal) silicon dioxide in dried or, for preference, hydrated form, three-layer clay materials such as smectite, in particular synthetic smectites with triochahedric co-ordinated cations, manufactured from magnesium silicates and alkali cations, or suitable colloidal magnesium-aluminium silicates; or synthetic hectorite clays such as Laponite® Laporte, London, Great Britain);
or mixtures of two or more of these components.

Particularly preferred are the "special organic gel formers" referred to under (iii).

The gel formers are present for preference in a proportion by weight, related to the finished preparation, from 0.1 to 15 % by weight, for preference (in particular in the case of organic gel formers) from 0.2 to 10 % by weight, such as, especially, from 0.25 to 4 % by weight.

The preparation can also include other additives selected from the group consisting of binders in particular, surface-active substances ("surfactants"), preservation agents, colouring agents, flavouring substances, pH regulators and regulators of osmotic activity.

Binders (in addition to the gel formers) may be, for example, dextrines, hydroxypropyl cellulose, hydroxypropyl methyl cellulose, polyvinyl pyrrolidon, gelatines, pectin, starch, or also sodium carboxymethyl cellulose, which in part also have gel-forming properties. These may be present in proportions of, for preference, up to 10 % by weight, for preference from 0.1 to 3 % by weight, related to the finished preparation.

Surfactants may be present in the preparation in quantities of up to 2 % by weight, for example from 0.2 to 0.8 % by weight. For preference, anionic detergents, such as sulphonates, are used, e.g. alkylbenzol sulphonates, alkanesulphonates, Alfa-olefin sulphonates, 2-acyloxy-alkan-1-sulphonates, or fatty acid monoglyceride sulphates; sulphates, such as alkyl sulphates, polyoxyethyl sulphates, fatty acid monoglyceride sulphates, or the salts thereof, for example with alkali metals, ammonia or ammonium compounds; cationic detergents, e.g. quaternary ammonium compounds, such as alkylaryiether dimethylbenzalkonium halogenides, alkyltrimethyl ammonium chlorides, alkyldimethyl ethylammonium halides, alkylpyridinium halogenides, alkyldimethyl ethylammonium halides, alkylpyridinium halogenides, alkylimidazolium halogenides, alkyldimethyl dichlorobenzyl ammonium halogenides, acylcolamonoformylinether pyridinium halogenide or alkylarylmethyl pyridinium halogenides; non-ionic detergents, such as polyethylene oxide condensates of alkylphenolene, ethylene oxide condensation products, derived from the reaction of ethylene oxide with the product from propylene oxide and ethylene diamine, the condensation products of aliphatic alcohols with ethylene oxide, or amide detergents which contain an ammonium, monoethanolamino, diethanolamino, or other alkanolamide group; zwitterionic detergents, such as aliphatic quaternary compounds containing ammonium and phosphonium or sulphonium groups; or amphoteric detergents, such as aliphatic secondary or tertiary amines. Preferred are the surface-active compounds referred to in WO 94/05330, as a result of which this application is incorporated here in this respect by reference.

Preservation agents are in particular complex formers, such as ethylenediamine tetraaceticacid, antioxidants such as ascorbic acid, butylated hydroxyanisol (BHA) or butylated hydroxytoluene (BHT) or other preservation agents such as methyl, ethyl, propyl, or butyl esters of 4-hydroxy-benzoic acid (Parabene), benzoic acid, cetrimide, cetyltrimethyl ammonium chloride, sorbinic acid or thiomersal, or the like. Preservation agents may be present, for example, in weight proportions (related to the finished preparation) of up to 1 % by weight, and in particular in the range from 0.005 to 0.5 % by weight. Preferably, preservation agents are omitted in order to avoid negative effects on the self-purifying mechanisms of the nasal mucosa.

As colouring agents, use may be made, for example, of toxicologically accetable substances such as beta-cerotin, Erythrosin, Gelborange S, Indigotin, or Tartrazin. By the addition of colouring agents of this type, can be examined, for example, to determine whether the preparations according to the invention are distributed over the entire nasal mucous membrane. Colouring agents may be present, for example, in the weight proportions (related to the finished preparation) of up to 1 % by weight, and in particular from 0.001 to 0.2 % by weight.

Flavouring substances are for preference not among the flavouring substances which belong to the etheric oils or plant extracts, but such as fruit flavouring substances (e.g. fruit esters) or vanillin, which may be present, for example, as up to 1 % by weight of the finished preparation, and in particular between 0.001 and 0.5 % by weight.

As pH regulators, use may be made of buffer additives, e.g. phosphate buffers or buffers on a citric acid basis. For preference these serve to adjust the pH value to between 3 and 9, and in particular between 5 and 8, and may be present in concentrations of up to, in particular, less than 120 mM, for example 20 to 100 mM.

As regulators of osmotic activity, use may be made, for example, of sugars or sugar alcohols (which can be used at the same time as additives) or, in particular, sodium chloride, for example (in particular for sodium chloride) in a concentration of up to 0.9 % (% by weight).

Alkyl (in case of sulphonates, alkan) has for preference 1 to 30, for example 1 to 18, carbon atoms, and can be unbranched or single-branched or multiple-branched, provided that sufficient carbon atoms are present. Aryl has for preference 6 to 14, and in particular 6 to 10, ring carbon atoms, and has a single ring or a double or multiple ring, and is unsubstituted or single-substituted or muftiple-substituted, in particular by alkyl, such as methyl or ethyl, carboxy, esterified carboxy, halogen, such as fluorine, chlorine, bromine or iodine, or also other substitutents; phenyl or naphthyl is preferred, unsubstituted or substituted as above. Acyl is for preference alkylcarbonyl or hydroxyalkylcarbonyl, in each case defined with alkyl as above, arylcarbonyl, for preference with aryl as defined above, or arylalkylcarbonyl, defined with aryl and alkyl as above.

If salt-forming groups (anionic, such as carboxy, sulphonyl, or sulphate) or cationic groups (such as amino) are present in the components or active substances referred to above, they may be also be present (in whole or in part) as (especially pharmaceutically acceptable) salts, in the case of anionic groups, for example, as alkali metal salts, alkaline-earth metal salts, ammonium salts, zinc salts, or tin salts, and in the case of cationic groups, for example as salts of organic or inorganic acids, such as of hydrogen halides hydrocarbons, sulphuric acid, phosphoric acid, organic sulphones or sulphates or carboxylic acids, such as acetic acid, or, if both anionic and cationic groups are present, as inner salts.

The person skilled in the art is aware, or can determine in a simple experimental manner, which of the components referred to can be combined with one another to form a preparation according to the invention, without mutually negative influences arising (precipitation, absence of thixotropic properties, or the like).

The term "therapeutic treatment", of the human in particular, also includes prophylaxis and the alleviation of symptoms, although not cosmetic treatments. This relates to therapeutic treatment, and in particular prophylaxis or alleviation, of disorders of the upper nasal passages, in particular of the nasopharynx (hereinbefore and hereinafter "nasal disorders), and in particular for the therapy and prophylaxis of allergic processes located therein, such as, in particular, allergic rhinitis, and especially pollinosis (for preference, since with low night-time exposure to pollen, treatment during the day is sufficient and also only requires seasonal therapy), as well as house dust or animal hair allergy.

Thixotropic gels are formulated in particular as nasal drops (usually in bottles with pipettes) or, for preference, as nasal sprays. For nasal sprays, portable spray devices are used in particular.

Examples of suitable (for preference activated by finger pressure) portable spray devices (nasal dispensers), which comprise a storage container, a spray pump, and for preference means for nebulization (if desired, also loose stirring elements, such as small metal pellets or rods, which contribute to the shaking up effect), are known, for example from German Patent DE 19 542 959, which describes both distribution pumps as well as simple squeeze bottles in combination with a storage container. Pre-compression pumps, such as are described in WO 98/00178, can also be used, such as the VP7/100S pump (Perfect Valois VP7), from Valois SA, France. Very well suited are also the "3-K Pumpe" (3-K Pump) or the COMOD system from AeroPump GmbH, Hofheim/Taunus, Germany. These types of pumps, especially in view of silver components, allow to avoid preservation agents. For preference, the dosage unit amounts to 1 to 50 ml, in the case of portable storage containers for nasal sprays for preference 3 to 30 ml, in particular 5 to 20 ml. The invention relates in particular to a thixotropic preparation, free of active substances according to the invention, inside a portable spray device of this type.

Administration can be effected in any desired manner. In the case of nasal administration, the quantity suitable for treatment of the disorders referred to, administered per spray burst, in particular the quantity of the preparation released with a nasal spray per spraying action, is for preference between 3 and 200 µl, and in particular between 50 and 150 µl, e.g. approximately 140 µl.

Administration takes place once or for preference several times daily, in particular at intervals of a few hours, for example at intervals of 1 to 6, and in particular of 2 to 4 hours; for preference starting directly after waking, and, if protection is also required at night, for the last time immediately before going to sleep.

The thixotropic preparations free of organic substances are manufactured according to conventional and known methods, which include in particular the mixing of the components, if required step by step and/or under movement and/or heating of the fluid, for example by stirring. A start is made for preference with sterile initial components and work is carried out under sterile conditions; incomplete interim stages, which do not yet contain all the components, are sterilised and/or the preparations are subjected at the end to a sterilisation procedure by suitable usual methods. The determination of the thixotropy is effected as described above, and a check can therefore easily be carried out as to whether the mixtures manufactured with the above components fall among the thixotropic gels to be used according to the invention. The enumeration of the components (A) to (C) upwards or downwards is not to be understood such that these components must be added one after another in sequence, and in particular not that water and the additives of component A are mixed separately; rather, the components can be mixed in any conceivable sequence, in particular the customary sequence and manner, for the manufacture of preparations according to the invention.

Use for the manufacture of a product for the therapeutic treatment of nasal disorders comprises in particular the appropriate procurement of the product (e.g. as a pharmaceutical product in the broader or narrower sense); in other words, the manufacture of the preparation, its introduction in particular into the spray device, its packing, and the corresponding provision of instructions for use in therapeutic treatment, for example by way of a package insert and/or printings on the package.

Preferred embodiments of the invention are described hereinafter, whereby, whenever mention is made of a "preparation", this means a thixotropic preparation, free of active substances, and, whenever the use of a preparation of this nature is referred to, this means use for the manufacture of a product for therapeutic treatment as referred to, unless defined otherwise.

The invention relates to the use of a preparation for the therapeutic treatment of disorders of the nose, especially of allergic rhinitis and of pollinosis.

The preparation used comprises for preference (A) an aqueous basis, in particular in a proportion of 10 to 80 % by weight, for preference from 30 to 70, more preferably from 40 to 60 % by weight, related to the finished preparation, whereby in the aqueous phase, as further water-soluble component, one or more additives are present in the form of one or more di- or polyols, especially glycerine, in such a quantity that the preparation contains a total of 0.1 to 10 % by weight thereof; (B) an oily component, in particular of hydrocarbons, such as, in particular, paraffin, one or more vegetable oils, or mixtures thereof, in particular low-viscosity paraffin and/or castor oil, whereby the oily component amounts to 10 to 80 % by weight, for preference 40 to 70, of the preparation; and (C) one or more gel formers for thixotropic gels, selected from organic suspension media, such as (i) polysaccharides, (ii) polymer compounds not belonging to the polysaccharides, or, especially, (iii) special organic gel formers, such as oxyethylenated polyol fatty acid esters, preferably alkyl-, especially methyl-monosaccharide-C₈-C₂₄ fatty acid esters or mixtures thereof, alone or in a mixture with corresponding non-oxyethylenated polyol fatty acid esters, preferably alkyl-, especially methyl monosaccharide-C₆-C₂₄ fatty acid esters, or mixtures thereof, in particular methylglucoside sesquistearate (= mixture of the monostearate and distearate), oxyethylenated with preferably 2 to 50, in particular 20 mol ethylene oxide, or a mixture thereof with methylglucoside sesquistearate, whereby the gel formers comprise a total of 0.1 to 15 % by weight, for preference 0.2 to 10 % by weight, in particular 0.25 to 4 %, of the complete preparation; in addition, further additives selected from the group consisting of binders up to 10 % by weight, in particular 0.1 to 3 % by weight, surfactants up to 1 % by weight, in particular 0.2 to 0.8 % by weight, preservation agents up to 1 % by weight, in particular from 0.005 to 0.5 % by weight, oolouring agents up to 1 % by weight, in particular from 0.001 to 0.2 % by weight, flavouring substances up to 1 % by weight, in particular from 0.001 to 0.5 % by weight, pH regulators in 120 nM or less concentration), and regulators of osmotic activity up to 0.9 % by weight, may be present in a less preferred embodiment of the invention, whereby, if salt-forming groups are present in the components, these may also be present (in whole or in part) as salts; and whereby, in the use of a particularly preferred preparation, only the components listed under (A), (B), and (C) are present (and/or pharmaceutically acceptable salts thereof).

The used preparations are in particular to thixotropic nasal drops or especially a nasal spray, for preference in a portable spray device, for preference a nasal dispenser, which comprises a storage container, a spray pump, and a device for nebulization.

A nasal spray, which is used for preference several times a day, in particular beginning shortly after waking and last used immediately before going to sleep, at intervals of several hours, in particular from 1 to 6, for preference from 2 to 4 hours, and for preference in a volume of between 3 and 500 µl, in particular between 50 and 150 µl, per nostril.

Especially preferred embodiments of the invention are also provided in the claims or in particular in the examples.

Examples: The following examples serve as illustrations of the invention, without restricting the scope thereof.

### Example 1: Thixotropic nose gel containing paraffin oil

A thixotropic gel is manufactured with the mixing of the components according to standard methods; see Rudolf Voigt, "Pharmazeutische Technologie" (Pharmaceutical Technology), Deutscher Apotherkerverlag (publishing house), 2000. In brief, the stirring process is pursued in a homogenizer for a short period of time at high speed (3000 to 6000 revolutions per minute, which leads to the formation of gel with cross-linking), with the result that the following composition of the resultant gel is obtained:

| Constituent class | Constituent | Proportion % by weight |
|---|---|---|
| Oily constituent | Paraffin oil. | 44.0 % |
| Gel formers | Glucate SS | 1.5% |
| | Glucamate SSE | 1.5% |
| Additive | Glycerine | 2.5 % |
| Basis | Water | 49.5 % |

Glucate SS is methyl glucoside sesquistearate. Glucamate SSE 20 is a methyl glucoside sesquestearate, which is polyoxyethylenated with 20 mol ethylene oxide. Both are available from Amerchol Corp., Edison, NJ, USA (now Union Carbide).

The paraffin oil is a low-viscosity paraffin oil (paraffinum perliquidum) according to the Europaean Pharmacopoea. The preparation is suitable for use as nasal drops, but is used in particular as nasal spray.

### Example 2: Thixotropic nasal gel containing castor oil

A thixotropic gel is manufactured with the mixing of the components according to the standard method as referred to under Example 1, so that the following composition of the resultant gel is obtained:

| Constituent class | Constituent | Proportion % by weight |
|---|---|---|
| Oily constituent | Castor oil | 44.0 % |
| Gel formers | Glucate SS | 1.5% |
| | Glucamate SSE | 1.5% |
| Additive | Glycerine | 2.5 % |
| Basis | Water | 49.5 % |

The castor oil is a product according to the Europaean Pharmacopoea. The preparation is suitable for use as nasal drops, but is also used in particular as a nasal spray.

### Example 3: Thixotropic preparation containing sodium cromoglycate

By analogy with the foregoing examples, a preparation containing active substance is manufactured making use of the components referred to therein, as well as an additional 2 % by weight sodium cromoglycate.

### Example 4: Nasal spray

One of the gels described in Examples 1 to 3 is filled into a conventional spray device (optionally, also equipped with a metal pellet which is freely movable under shaking, to promote peptisation in the storage chamber). The spraying device is a combination of storage container with 20 ml content and a dosage pump with a nose adapter for nebulization. A suitable spray device of this type, which also has the advantage that no anti-microbial active substances are required in the compositions used for filling, since silver is provided in the outlet nozzle for the disinfection of the spray residue which may flow back, is one of the models with the designation "3-K Pumpe" (3-K pump) or COMOD system (AeroPump GmbH, nebulization pumps, Hofheim/Taunus, Germany); in this case, 140 µl is administered per spray action. After brief shaking, the gel present in the storage container is liquified (sol state), and can be applied as a spray mist with no trouble, using the administration pump located on the storage container. Once the mist impinges on the surface of the mucosa, the settlement phase beings, and the thixotropic gel solidifies again and forms a film on the surface of the nasal mucous membrane. This forms a layer which is permeable for allergens only with difficulty or is impermeable for them, with the result that they do not come in contact with mast cells.

### Example 5: Usefulness of a manufactured product in patients

In the scope of a field study, the efficacy of a nasal spray spraying device according to Example 4, containing a thixotropic gel according to Example 1, is tested on patients with pollinosis. The study is conducted on 25 patients. In the first instance they are asked about the seasonal occurrence of symptoms daily for a period of one week, for example as to whether they suffer from swelling of the nose, secretion from the nasal mucous membrane, sneezing, or the like. The same patients then use a nasal spray according to Example 3, with the gel according to the invention from Example 1. The administration is effected for the first time after getting up in the morning, and thereafter at intervals of a few hours (e.g. 3-4), until going to sleep. After shaking the gel to cause it to change to the sol state, a spray volume of 100 µl is administered to each nostril with the head indined slightly backwards. During the week of treatment, the patients are asked daily about their symptoms, for example as to whether they are suffering from swelling of the nose, increased secretion from the nasal mucous membrane, sneezing, conjunctivitis or the like. Most of the patients present symptoms which are clearly reduced in comparison with the week free of therapy, and also present a subjective improvement in their symptoms.

### Example 6: Further example of usefulness of a manufactured product in patients

By analogy for example with Example 5, instead of the gel from Example 1, the gel from Example 2 or 3 is used on patients as a nasal spray.

## Claims

1. The use of a thixotropic preparation, free of active substances, for the manufacture of a pharmaceutical product without the addition of pharmaceutically active substances for the therapeutic treatment of nasal disorders, where the term "therapeutic treatment" also includes prophylaxis,
where the thixotropic preparation consists of
(A) an aqueous basis, whereby the aqueous phase can contain one or more additives in the form of one or more di- or polyols;
(B) an oily component; and
(C) one or more gel formers for thixotropic gels;
with or without the presence of further additives selected from the group consisting of binders, surfactants, preservation agents, colouring agents, flavouring substances, pH regulators, and regulators of the osmotic activity,
whereby, if salt-forming groups are present in the components, these may also be present in whole or in part as salts.

2. The use according to claim 1 where the disorder to be treated is allergic rhinitis.

3. The use according to claim 1 or claim 2 where the disorder to be treated is pollinosis.

4. The use according to any one of claims 1 to 3 where the thixotropic preparation consists of
(A) an aqueous phase in a proportion of 10 to 80 % by weight, for preference from 30 to 70 % by weight, more preferably from 40 to 60 % by weight and one or more di- or polyols, especially glycerine, in such a quantity that the preparation contains a total of 0.1 to 10 % by weight thereof;
(B) an oily component, preferably of hydrocarbons, vegetable oils or hydrated, polyoxyethylated or polyoxy- or hydrated polyoxy or fractionated derivatives thereof in a proportion by weight in the finished preparation from 10 to 80 % by weight, in particular from 40 to 70 % by weight; and
(C) at least one gel forming agent for thixotropic gels whereby the gel forming agent(s) are present, related to the finished preparation, in from 0.1 to 15 % by weight, for preference from 0.2 to 10 % by weight, especially from 0.25 to 4 % by weight;
with or without the presence of further additives selected from the group consisting of related to the finished preparation, binders up to 10 by weight, surfactants up to 1 % by weight, preservation agents up to 1 % by weight, colouring agents up to 1 % by weight, flavouring substances up to 1 % by weight, pH regulators in 120 mM or less concentration, and up to 0.9 % by weight regulators of the osmotic activity,
whereby, if salt-forming groups are present in the components, these may also be present (in whole or in part) as salts.

5. The use according to any one of claims 1 to 4 wherein
(A) the aqueous basis is water with glycerine;
(B) the oily component is a hydrocarbon or a vegetable oil or the hydrated, polyoxyethylated, polyoxy- or hydrated polyoxyderivative derivatives thereof; or mixtures of two or more of these components;
(C) the gel-forming agent for thixotropic gels is selected from the group consisting of organic suspension media and inorganic suspension media, or mixtures of two or more of these components;
with or without the presence of further additives selected from the group consisting of binders, surfactants, preservation agents, colouring agents, flavouring substances, pH regulators, and regulators of the osmotic activity,
whereby, if salt-forming groups are present in the components, these may also be present (in whole or in part) as salts.

6. The use according to any one of claims 1 to 5 where only the components listed under (A), (B) and (C) and/or salts thereof are present.

7. The use according to any one of claims 1 to 6 where the gel former for thixotropic gels (C) is selected from polyoxyethylated polyol fatty acid esters, for preference alkyl-, especially methyl-, monosaccharide-C₆-C₂₄ fatty acid esters, or mixtures thereof, alone or in a mixture with corresponding non-oxyethylenated polyol fatty acid esters, or mixtures thereof; in particular methylglucoside sesquistearate, oxyethylated with 2 to 50 mol, especially 20 mol, ethylene oxide or a mixture thereof with methylglucoside sesquistearate; and further inorganic suspension media.

8. The use according to any one of claims 1 to 7 where the thixotropic preparation, free of active substances, has the following composition: Paraffin oil 40 to 50 % by weight, for preference 44 % by weight; methylglucoside sesquistearate, oxyethenylated with 20 mmol ethylene oxide 0.5 to 5 % by weight, for preference 1.5 % by weight; methylglucoside sesquistearate 0.5 to 5 % by weight, for preference 1.5 % by weight; glycerine 0.5 to 5 % by weight, for preference 2.5 % by weight; and water 40 to 60 % by weight, for preference 49.5 % by weight.

9. The use according to any one of claims 1 to 7 where the thixotropic preparation, free of active substances, has the following composition: Castor oil 40 to 50 % by weight, for preference 44 % by weight; methylglucoside sesquistearate, oxyethenylated with 20 mmol ethylene oxide 0.5 to 5 % by weight, for preference 1.5 % by weight; methylglucoside sesquistearate 0.5 to 5 % by weight, for preference 1.5 % by weight; glycerine 0.5 to 5 % by weight, for preference 2.5 % by weight; and water 40 to 60 % by weight, for preference 49.5 % by weight.

10. The use according to any one of claims 1 to 9 wherein the pharmaceutical product is a nasal spray.

11. The use according to claim 2, or any one of claims 3 to 10 as far as they refer to claim 2, where the effect against the allergens is based on a mechanical barrier function.

## Patentansprüche

1. Verwendung eines thixotropen Präparats, das frei von Wirkstoffen ist, zur Herstellung eines pharmazeutischen Produkts ohne die Zugabe von pharmazeutischen Wirkstoffen zur therapeutischen Behandlung von Nasenstörungen, wobei der Begriff "therapeutische Behandlung" auch Prophylaxe einschließt,
wobei das thixotrope Präparat aus Folgendem besteht:
(A) einer wässrigen Basis, wobei die wässrige Phase ein oder mehrere Additive in Form von einem oder mehreren Polyolen enthalten kann;
(B) eine Ölkomponente; und
(C) einen oder mehrere Gelbildner für thixotrope Gele;
mit der oder ohne die Gegenwart von weiteren Additiven ausgewählt aus der Gruppe bestehend aus Bindemitteln, oberflächenaktiven Mitteln, Konservierungsmitteln, Farbmitteln, Geschmackstoffen, pH-Regulatoren und Regulatoren der osmotischen Aktivität,
wobei, falls salzbildende Gruppen in den Komponenten vorliegen, diese auch gänzlich oder teilweise als Salze vorliegen können.

2. Verwendung nach Anspruch 1, wobei die zu behandelnde Störung allergische Rhinitis ist.

3. Verwendung nach Anspruch 1 oder 2, wobei die zu behandelnde Störung Heufieber ist.

4. Verwendung nach einem der Ansprüche 1 bis 3, wobei das thixotrope Präparat aus Folgendem besteht:
(A) einer wässrigen Phase mit einem Anteil von 10 bis 80 Gew.-%, vorzugsweise 30 bis 70 Gew.-%, stärker bevorzugt 40 bis 60 Gew.-% und einem oder mehreren Di- oder Polyolen, insbesondere Glycerin in einer derartigen Menge, dass das Präparat insgesamt 0,1 bis 10 Gew.-% davon enthält;
(B) einer Ölkomponente, vorzugsweise aus Kohlenwasserstoffen, Pflanzenölen oder hydratisierten, polyoxyethylierten oder Polyoxy- oder hydratisierten Polyoxy- oder fraktionierten Derivaten davon in einem Gewichtsanteil im fertigen Präparat von 10 bis 80 Gew.-%, insbesondere von 40 bis 70 Gew.-%; und
(C) mindestens einem gelbildenden Mittel für thixotrope Gele, wobei das (die) gelbildende(n) Mittel in Bezug auf das fertige Präparat mit 0,1 bis 15 Gew.-%, vorzugsweise 0,2 bis 10 Gew.-%, insbesondere 0,25 bis 4 Gew.-% vorliegt (vorliegen);
mit der oder ohne die Gegenwart von weiteren Additiven ausgewählt aus der Gruppe bestehend aus bis zu 10 Gew.-% Bindemitteln, bis zu 1 Gew.-% oberflächenaktiven Mitteln, bis zu 1 Gew.-% Konservierungsmitteln, bis zu 1 Gew.-% Farbmitteln, bis zu 1 Gew.-% Geschmackstoffen, pH-Regulatoren mit 120 mM oder einer geringeren Konzentration und bis zu 0,9 Gew.-% Regulatoren der osmotischen Aktivität, in Bezug auf das fertige Präparat,
wobei, falls salzbildende Gruppen in den Komponenten vorliegen, diese auch (gänzlich oder teilweise) als Salze vorliegen können.

5. Verwendung nach einem der Ansprüche 1 bis 4, wobei
(A) die wässrige Basis Wasser mit Glycerin ist;
(B) die Ölkomponente ein Kohlenwasserstoff oder ein Pflanzenöl oder die hydratisierten, polyoxyethylierten oder Polyoxy- oder hydratisierten Polyoxy- oder fraktionierten Derivate davon oder Gemische aus zwei oder mehreren dieser Komponenten ist;
(C) das gelbildende Mittel für thixotrope Gele ausgewählt ist aus der Gruppe bestehend aus organischen Suspensionsmedien und anorganischen Suspensionsmedien oder Gemischen aus zwei oder mehreren dieser Komponenten;
mit der oder ohne die Gegenwart, von weiteren Additiven ausgewählt aus der Gruppe bestehend aus Bindemitteln, oberflächenaktiven Mitteln, Konservierungsmitteln, Farbmitteln, Geschmackstoffen, pH-Regulatoren und Regulatoren der osmotischen Aktivität,
wobei, falls salzbildende Gruppen in den Komponenten vorliegen, diese auch (gänzlich oder teilweise) als Salze vorliegen können.

6. Verwendung nach einem der Ansprüche 1 bis 5, wobei nur die unter (A), (B) und (C) aufgezählten Komponenten und/oder Salze davon vorliegen.

7. Verwendung nach einem der Ansprüche 1 bis 6, wobei der Gelbildner für thixotrope Gele (C) ausgewählt ist aus polyethoxylierten Polyolfettsäureestern, vorzugsweise Alkyl-, insbesondere Methylmonosaccharid-C₆-C₂₄-Fettsäureestern oder Gemischen davon, allein oder in einem Gemisch mit entsprechenden nicht-oxyethylenierten Polyolfettsäureestern oder Gemischen davon; insbesondere Methylglucosidsesquistearat, das mit 2 bis 50 mol, insbesondere 20 mol Ethylenoxid oxyethyliert ist, oder einem Gemisch davon mit Methylglucosidsesquistearat, und weiteren anorganischen Suspensionsmedien.

8. Verwendung nach einem der Ansprüche 1 bis 7, wobei das thixotrope Präparat, das frei von Wirkstoffen ist, die folgende Zusammensetzung aufweist: 40 bis 50 Gew.-%, vorzugsweise 44 Gew.-% Parafinöl; 0,5 bis 5 Gew.-%, vorzugsweise 1,5 Gew.-% Methylglucosidsesquistearat, das mit 20 mmol Ethylenoxid oxyethyleniert ist; 0,5 bis 5 Gew.-%, vorzugsweise 1,5 Gew.-% Methylglucosidsesquistearat; 0,5 bis 5 Gew.-%, vorzugsweise 2,5 Gew.-% Glycerin und 40 bis 60 Gew.-%, vorzugsweise 49,5 Gew.-% Wasser.

9. Verwendung nach einem der Ansprüche 1 bis 7, wobei das thixotrope Präparat, das frei von Wirkstoffen ist, die folgende Zusammensetzung aufweist: 40 bis 50 Gew.-%, vorzugsweise 44 Gew.-% Rhizinusöl; 0,5 bis 5 Gew.-%, vorzugsweise 1,5 Gew.-% Methylglucosidsesquistearat, das mit 20 mmol Ethylenoxid oxyethyleniert ist; 0,5 bis 5 Gew.-%, vorzugsweise 1,5 Gew.-% Methylglucosidsesquistearat; 0,5 bis 5 Gew.-%, vorzugsweise 2,5 Gew.-% Glycerin und 40 bis 60 Gew.-%, vorzugsweise 49,5 Gew.-% Wasser.

10. Verwendung nach einem der Ansprüche 1 bis 9, wobei das pharmazeutische Produkt ein Nasenspray ist.

11. Verwendung nach Anspruch 2 oder einem der Ansprüche 3 bis 10, sofern sie Anspruch 2 betreffen, wobei die Wirkung gegen die Allergene auf einer mechanischen Sperrfunktion basiert.

## Revendications

1. Utilisation d'une préparation thixotrope, exempte de substances actives, pour la fabrication d'un produit pharmaceutique sans adjonction de substances actives sur le plan pharmaceutique et destinée au traitement thérapeutique de pathologies nasales, le terme "traitement thérapeutique" comprenant également la prophylaxie, dans laquelle la préparation thixotrope se compose
(A) d'une base aqueuse, ladite base aqueuse pouvant contenir un ou plusieurs additif(s) sous la forme d'un ou de plusieurs diol(s) ou polyol(s) ;
(B) d'un composant huileux ; et
(C) d'un ou plusieurs agent(s) gélifiant(s) pour gels thixotropes;
avec ou sans additifs supplémentaires choisis dans le groupe constitué de liants, de tensioactifs, de conservateurs, de colorants, de substances aromatisantes, de régulateurs de pH et de régulateurs de l'activité osmotique, dans laquelle, si des groupes salificateurs sont présents dans les composants, ceux-ci peuvent également se présenter, pour tout ou partie, sous la forme de sels.

2. Utilisation selon la revendication 1, dans laquelle la pathologie à traiter est la rhinite allergique.

3. Utilisation selon la revendication 1 ou 2, dans laquelle la pathologie à traiter est la pollinose.

4. Utilisation selon l'une quelconque des revendications 1 à 3, la préparation thixotrope se composant
(A) d'une phase aqueuse dans une proportion de 10 à 80 % en poids, de préférence de 30 à 70 % en poids, de façon plus préférable de 40 à 60 % en poids, et d'un ou plusieurs diol(s) ou polyol(s), en particulier la glycérine, dans une quantité telle que la préparation en totalité en contient 0,1 à 10 % en poids ;
(B) d'un composant huileux, de préférence d'hydrocarbures, d'huiles végétales ou de leurs dérivés hydratés, polyoxyéthylés, ou polyoxy ou polyoxy-hydratés ou fractionnés, dans une proportion pondérale rapportée à la préparation finie comprise entre 10 et 80 % en poids, en particulier entre 40 et 70 % en poids ; et
(C) d'au moins un agent gélifiant pour gels thixotropes, le ou les agent(s) gélifiant(s) étant présent(s) à raison de 0,1 à 15 % en poids, de préférence de 0,2 à 10 % en poids, en particulier de 0,25 à 4 % en poids de la préparation finie ;
avec ou sans additifs supplémentaires choisis dans le groupe constitué de liants jusqu'à 10 % en poids, de tensioactifs jusqu'à 1 % en poids, de conservateurs jusqu'à 1 % en poids, de colorants jusqu'à 1 % en poids, de substances aromatisantes jusqu'à 1 % en poids, de régulateurs de pH jusqu'à une concentration inférieure ou égale à 120 mM et de régulateurs de l'activité osmotique jusqu'à 0,9 % en poids, rapporté à la préparation finie où, si des groupes salificateurs sont présents dans les composants, ceux-ci peuvent également se présenter (pour tout ou partie) sous la forme de sels.

5. Utilisation selon l'une quelconque des revendications 1 à 4, où
(A) la base aqueuse est constituée d'eau et de glycérine ;
(B) le composant huileux est un hydrocarbure ou une huile végétale ou l'un de ses dérivés hydratés, polyoxyéthylés ou polyoxy ou polyoxy-hydratés ou un mélange de deux ou de plusieurs de ces composants;
(C) l'agent gélifiant pour gels thixotropes est choisi dans le groupe constitué de milieux de suspension organiques et de milieux de suspension inorganiques, ou de mélanges de deux ou plusieurs de ces composants;
avec ou sans additifs supplémentaires choisis dans le groupe constitué de liants, de tensioactifs, de conservateurs, de colorants, de substances aromatisantes, de régulateurs de pH et de régulateurs de l'activité osmotique,
où, si des groupes salificateurs sont présents dans les composants, ceux-ci peuvent également se présenter (pour tout ou partie) sous la forme de sels.

6. Utilisation selon l'une quelconque des revendications 1 à 5, où seuls sont présents les composants listés en (A), (B) et (C) et/ou leurs sels.

7. Utilisation selon l'une quelconque des revendications 1 à 6, où l'agent gélifiant pour gels thixotropes (C) est choisi parmi des esters d'acides gras de polyols polyoxyéthylés, de préférence des esters d'acides gras de monosaccharides en C₆ à C₂₄, de préférence de type alkyle et en particulier méthyle, ou leurs mélanges, seuls ou en mélange avec les esters d'acides gras de polyols non oxyéthylénés correspondants ou leurs mélanges ; en particulier le sesquistéarate de méthylglucoside oxyéthyléné avec 2 à 50 moles, en particulier 20 moles d'oxyde d'éthylène ou un mélange de celui-ci avec du sesquistéarate de méthylglucoside ; et des milieux de suspension inorganiques supplémentaires.

8. Utilisation selon l'une quelconque des revendications 1 à 7, dans laquelle la préparation thixotrope, exempte de substances actives, présente la composition suivante : 40 à 50 % en poids d'huile de paraffine, de préférence 44 % en poids ; 0,5 à 5 % en poids de sesquistéarate de méthylglucoside oxyéthyléné avec 20 mmoles d'oxyde d'éthylène, de préférence 1,5 % en poids ; 0,5 à 5% en poids de sesquistéarate de méthylglucoside, de préférence 1,5 % en poids ; 0,5 à 5 % en poids de glycérine, de préférence 2,5 % en poids ; et 40 à 60 % en poids d'eau, de préférence 49,5 % en poids.

9. Utilisation selon l'une quelconque des revendications 1 à 7, où la préparation thixotrope, exempte de substances actives, présente la composition suivante : 40 à 50 % en poids d'huile de ricin, de préférence 44 % en poids ; 0,5 à 5 % en poids de sesquistéarate de méthylglucoside oxyéthyléné avec 20 mmoles d'oxyde d'éthylène, de préférence 1,5 % en poids ; 0,5 à 5 % en poids de sesquistéarate de méthylglucoside, de préférence 1,5 % en poids ; 0,5 à 5 % en poids de glycérine, de préférence 2,5 % en poids ; et 40 à 60 % en poids d'eau, de préférence 49,5 % en poids.

10. Utilisation selon l'une quelconque des revendications 1 à 9, dans laquelle le produit pharmaceutique est un spray nasal.

11. Utilisation selon la revendication 2, ou selon l'une quelconque des revendications 3 à 10, dès lors qu'elles se rattachent à la revendication 2, dans laquelle l'effet anti-allergène repose sur une fonction de barrière mécanique.
